# EUROPEAN PATENT APPLICATION

(11) **EP 2 374 786 A1**
(43) Date of publication of application: **12.10.2011**
(21) Application number: 10159548.6
(22) Date of filing: 09.04.2010
(51) Int. Cl.: C07C 51/43, C07C 53/16

(54) **Purification of monochloroacetic acid rich streams**

(71) Applicant: GEA Niro PT B.V., 5221 EE's-Hertogenbosch (NL)
(72) Inventor: The designation of the inventor has not yet been filed
(74) Representative: Hatzmann, Martin

(57) **Abstract**

The invention relates to a method for the manufacture of a substantially pure MCAA by combining a suspension-based melt crystallization process with a subsequent separation of the pure MCAA crystals in a wash column.

## Description

### Technical Field

The invention relates to a method for the purification or recovery of monochloroacetic acid rich streams.

### Background of the invention

Monochloroacetic acid (commonly abbreviated as MCA or MCAA) is almost exclusively used as a chemical intermediate. The major industrial use accounting for 30-40% of its production, is in the manufacture of carboxymethyl cellulose (CMC). The sodium salt of CMC is made by reacting starch with MCAA in a strongly alkaline NaOH medium. The MCAA is usually used as an 80% solution.

MCAA is also used to make the important arylhydroxyacetic acid, methyl β-napthyloxyacetate and benazoline based herbicides, as well as the insecticides dimethoate, 2,4-dichlorophenoxyacetic acid (2,4-D) and dimethoate. MCAA is also used to make glycophosate.

Glycolic acid (and diglycolic acid) is prepared industrially by heating MCAA with an alkali hydroxide. Glycolic acid is used in textile printing, leather treatments and cleaners. Glycolic acid esters are used to make plasticizers.

Heating MCAA with sodium or potassium hydrogen sulphide gives thioglycolic acid. Thioglycolic acid and its derivatives are used in the production of hair cosmetics. The use of tin alkyl thioglycolates are increasing in importance as they replace more toxic lead based PVC thermal stabilizers.

In addition MCAA is used to make surfactants, particularly mild bentine amphoteric surfactants.

Because of its reactivity, MCAA is widely used in many other organic syntheses, as well. It reacts with alcohols to give alkoacetic acids. With ammonia and amines it gives a-amino acids and phenylglycine.

MCAA is a hygroscopic, white crystalline solid with a density of 1.58 kg/dm³. It is highly corrosive and toxic. It therefore requires careful handling in corrosion resistant equipment. A low operating temperature is an inherent process advantage.

Purified MCAA is typically sold in three different grades: (i) technical grade with a maximum impurity content of 0.5 wt.%, (ii) high purity grade with a maximum impurity content of 0.2 wt.%, and (iii) ultra pure grade with a maximum impurity content of 0.05-0.01 wt.%. Pharmaceutical grade MCAA contains a specified maximum of specific impurities, such as a maximum of 100 ppm of dichloro acetic acid.

The MCAA product is usually converted to a 100% flakes material. In addition, MCAA is also sold as molten liquid or as a bulk 80% solution in water or alcohol.

MCAA can be produced by the chlorination of acetic acid (most common route), the hydrolysis of trichloroethylene using sulfuric acid or the oxygenation of vinylidene chloride to chloroacetyl chloride, which is then reacted with water. All routes lead to a MCAA reach stream that contains varying amounts of impurities.

The high degree of purity required for most applications (> 99.5 wt% MCAA) calls for further purification. Various alternatives have been proposed in the prior art for purifying the crude melt.

Distillation is carried out in two stages. The first one removes volatile acetic acid and acetic anhydride which are returned to the chlorinator. Then pure MCAA is evaporated and is sent to product recovery. Since the boiling points of the three chlorinated acetic acids are so close, the di- and tri chloroacetic acids cannot be separated economically by normal distillation. Azeotropic distillation and extractive distillation have been suggested for separating dichloro acetic acid, but due to the very high energy consumption and other specific problems it is doubtful, whether these methods are used in commercial practice at all.

In US patent application US 2005/0272953 a reactive distillation process is proposed which the authors claim gives better control over chlorination, reducing the formation of DCA and is a more effective way of recycling the acetic anhydride/acetyl chloride catalyst. Disadvantages are high investment-, operation- and catalyst cost.

In European patent application EP 1 900 719 A1 it has been suggested to use a loop reactor for the selective catalytic hydrogenation of dichloro acetic acid to MCAA. This process works at temperatures of around 150 °C and a pressure of 3 bar and is dependent on the availability of hydrogen; it also features high catalyst cost. Care must be taken not to convert MCAA back to acetic acid. Aldehydes can form which color the white MCAA.

Another problem of all processes working at the vapor-liquid equilibrium is the high corrosiveness of the MCAA product which is becoming more pronounced at higher temperatures thereby calling for ultra expensive materials of construction.

The MCAA can also be purified by crystallization from the melt or solution. A number of solution technologies have been patented involving crystallization from water or chlorinated solvents.

The purification in all melt crystallization process is a result of the higher melting point of the α-modification of chloroacetic acid (m.p. = 63 °C). Until the eutectic point is reached, di- and trichloro acetic acids, as well as all other impurities are excluded from the MCAA crystal and therefore concentrated in the remaining liquid mother-liquor. The difficulty with melt crystallization is the recovery of an appropriate crystal product from the concentrated melt and its subsequent separation from such mother liquor. Melt crystallization is carried out either on stationary heat transfer surfaces upon which one massive crystal block is formed (layer crystallization) or in stirred suspension-based crystallizers. With the latter, small individual crystals are produced and then subsequently separated by centrifugation. The product is washed with water or acetic acid and discharged. Achievable purities are typically limited to 98-99 wt.% (in specific cases up to 99.5wt.%) and therefore not adequate for many of the above described applications.

In layer crystallization, the purification step is carried out by using cold fingers or other cooled surfaces. When a certain amount of the chloroacetic acid has crystallized onto the fixed heat exchanger surface, the process is stopped, the mother-liquor is drained; and the whole system is heated to remove the produced crystalline product from the heat exchanger surface.

US 5,756,840 describes a process for the preparation of a particularly pure MCAA, in which, in a first step, a hydrogenation of a mixture of mono- and dichloro acetic acid is carried out to a residual content of dichloro acetic acid of 400 to 600 ppm and, in a second step, this mixture is subjected to melt crystallization. Although in US 5,756,840 it is claimed that the process can be applied to mixtures containing up to 50 wt.% of dichloro acetic acid, such amounts of said impurity call for passing through the hydrogenation stage twice, prior to being treated by typically multistep batch-wise operated melt crystallization (with respective disadvantages as described below), a process which is not economic.

GB 1,275,231 describes a process for the preparation of a particularly pure MCAA from a reaction mixture by means of static melt crystallization in a crystallizer fitted with 89 tubular cooling fingers.

Discontinuous or semi-continuous static and dynamic layer crystallization processes as described in the above two patents are known from the prior art for the manufacture, purification and recovery of other chemical applications: e.g. acrylic acid, DMT, para- and meta- xylene, among others. Since the surface available for crystal growth equals the heat exchanger surface, such layer melt crystallization processes have (for economical reasons) to be operated at relatively high crystal growth rates between 10⁻⁵ m/s and 10⁻⁶ m/s. These high growth rates are not required by the system but generally dictated by the economics and required size of the equipment. Suspension based systems rely on the growth surface of billions of individual crystals which provide a massive cumulative surface and result in growth rates which are about a factor of 100 to 1,000 lower than the layer based system. The high growth rates found in most layer based systems lead to non-equilibrium crystal growth and finally results in impure crystal product. While the growing crystal lattice in such processes still consist of pure MCAA, the high growth rates entail that the crystals grow exhibits a dendrite like structure and mother liquor containing dichloro acetic acid, trichloro acetic acid and all other impurities becomes entrapped into this complex structure. In layer crystallization the crystal growth rates typically exceeds the diffusion rate of the impurities, which get concentrated in front of the growing crystal layer, back into the bulk solution; such a situation typically leads to a dynamic, non equilibrium controlled inclusion of impurities into the crystal lattice at such faster growth rates: the crystals grow in a dendrite like structure with inclusions of the high purity containing liquid close to the growth surface.

The separation of a single layer melt crystallization process can be enhanced by additional purification methods like sweating and washing: these methods offer an increase in purification efficiency at the expense of the product yield. If such a melt crystallization process is performed in an optimized way by a person skilled in the art, the solid phase can typically be 5 to 10 times purer than the liquid melt, in case additional sweating and/or washing is applied this purification ratio can be as high as 20.

In the several examples of US patent US 5,756,840 above as well as in the examples of GB 1,275,231 above, a purification ratio of only 4 to 5,8 is disclosed. This means that such layer crystallization process needs to be repeated several times in order to achieve commercially acceptable MCAA purities.

Apart from the obvious economical disadvantage related to such process repetitions, they drastically reduce the process yield and further increase energy consumption; the process is energy intensive and because of its discontinuous nature does not allow the crystallizer to run at a constant temperature. The process always cycles between a cooling phase to create the crystal layer and a heating phase to remove the crystallized product from the heat exchanger surface which involves wasted energy to heat and cool the mass of equipment. The lower process yield is due to the fact that only a certain portion can be crystallized out of a certain amount of feed. If, for example 50% of the initial mass is crystallized on the heat exchanger surface of the crystallizer and such process is repeated twice, the final product is only 25% of the initial amount of feed. The various intermediate fractions can also be subjected to the method of the above two inventions thereby increasing the yield again but requiring an even more complex process structure with a variety of multiple purity and recovery stages. The frequent repetition of multiple batch wise process steps with intermediate melting and recrystallization consumes much more energy than a single stage process.

### Summary of the present Invention

In general terms, the present invention is accordingly based on mitigating or overcoming the disadvantages arising out of the prior art. In particular, the invention shall make available a method for the manufacture of substantially pure MCAA, which does not use any solvent and which overcomes the disadvantages associated with frequent repetition of multiple batch crystallization steps, does not require excessive catalyst or other raw materials which require further processing or disposal as waste and works at moderate temperatures as to minimize corrosion problems and decrease the use of ultra expensive materials of construction.

The afore-mentioned problems are solved firstly by the subject matter of the claims herein below. Furthermore, the afore-mentioned problems are solved by a method comprising:
1. a continuous efficient suspension based melt crystallization process where the crystals are grown in a carefully under-cooled melt in a manner that spontaneous nucleation does not occur and in a way that the crystals (i) do not include any impurities and (ii) are suitable for the subsequent separation in a wash column.
2. separation of the produced MCAA crystals in a wash column and preferably a forced transport type of wash column, which provides a highly efficient countercurrent washing operation in a bed depth of 10 to 200 cm wherein the purity of the discharged MCAA product can be controlled by the intensity of washing. The wash liquid comprises molten MCAA crystals as purified in the said wash column. The wash column process can be operated with controlled losses of wash liquid or more preferably without the loss of any wash liquid.

Surprisingly it has been found that such method is suitable to manufacture a substantially pure MCAA product in a single stage continuous process at moderate temperatures while essentially retaining all impurities in the separated mother liquor.

### Brief description of the drawings

The present invention will be described, by way of example, with reference to the accompanying drawings in which:
Fig. 1 shows the phase diagram for MCAA and DCAA
Fig. 2 shows a crystallization system for carrying out the method of the present invention which system consists of a heat exchanger, a mixing vessel and a wash column
Fig. 3 shows as an preferred embodiment of the present invention a crystallization system consisting of a combined heat exchanger and mixing vessel connected to a wash column, and
Fig. 4 shows a system for carrying out the method of the present invention in accordance with Fig.2, but comprising an additional mixing vessel upstream of the wash column.

### Detailed description of the invention

In principle the novel method of this invention is suitable for all MCAA rich feeds and is especially useful for those feed streams as mentioned in this publication. All concentration figures and temperatures as presented in this invention are given for the purification of a specific MCAA rich feed, but may easily be adopted by a person skilled in the art to other feed solutions.

Frequently the MCAA rich feedstock contains between 80% and 99% of MCAA by weight. The remainder includes, for example, di chloro acetic acid, tri chloro acetic acid, hydroxilic impurities like water, and other impurities like acetic acid and sulfate.

The method of the present invention is capable to produce a substantially pure MCAA product suitable for the downstream synthesis of products as, amongst others, carboxymethyl cellulose, carboxymethyl starch, herbicides, insecticides, stabilizers for poly vinyl chloride, thioglycolic acid, glycolic acid and the remainder of products produced from its derivatives. Substantially pure MCAA can be obtained with the method of the present invention ranging from 98wt% MCAA to 99.99 wt.% MCAA and even higher purities would be achievable with the method of the present invention.

Suspension based crystallization provides a system for continuous operation. While such continuous operation is preferred, the process can also be operated batch wise based on manufacturing requirements. The large crystal mass of the suspended crystals provides a massive growth surface and allows for very slow and near ideal growth rates. It is preferred that the total available surface area for crystal growth in the suspension process of this method is around some 5,000 to 20,000 m² per m³ of the crystallizer volume. This far exceeds the growth surface typically available in layer type system which is generally limited to <100m² per m³ of system volume. The massive growth surface in suspension based crystallization allows that any given under-cooling is "absorbed" by such large growth surface and in turn results in extremely slow crystal growth rates in the range of 10⁻¹⁰ to 10⁻⁸ m/s. At the said slow growth rates the MCAA molecules are included into the highly ordered crystal lattice under near equilibrium conditions allowing new MCAA molecules sufficient time for the required diffusion from the homogeneously mixed bulk liquid mass through the boundary layer before they become incorporated into the crystal structure.

In the preparation of the MCAA suspension required according to the invention it is essential that the crystals are grown at the said slow growth rates in the under-cooled melt This can be accomplished in various commercially available scraped or un-scraped cooled stirred vessel crystallizers, as described for example in Melt Crystallization Technology; G.F. Arkenbout; Technomic Publishing Company Inc.; 1995. The scraped crystallizers have to be operated in a way to constantly sweep the heat exchanger surface in order to slightly under-cool the melt, but preventing a crystal layer to develop on the heat exchanger surface. Very generally, all suspension crystallizers which are stated in the above publication as prior art or which are listed in this invention are suitable to be applied for the novel method for the preparation of a MCAA suspension.

Suspension crystallization processes, including the crystallization of MCAA rich feeds, always involve both, nucleation and crystal growth. Such two mechanisms are a function of the under-cooling as well as other process parameter and system properties and finally determine the crystal size along with its distribution obtained with such crystallization processes. Two different types of nucleation are generally distinguished: primary- or spontaneous nucleation which suddenly starts, when the under-cooling exceeds a certain limit and secondary nucleation, which is a function of the under-cooling. The inventors found that it is important to avoid spontaneous nucleation at the start of the crystallization process, since this would result in the formation of a viscous inseparable mass of billions of extremely fines crystals suspended in the remaining mother liquor where such tiny crystals cannot be separated from the remaining mother liquor any more. In a specially preferred embodiment of the present invention a seeding procedure is applied in order to start the crystallization process in the absence of spontaneous nucleation, such seeding procedure comprising the steps of:
a) Adding the MCAA rich feed solution to a mixing vessel at a concentration such that, when cooled to a temperature between 48°C and 60°C said solution will be under-cooled with respect to the relevant MCAA molecule;
b) Cooling down said MCAA rich feed solution to a temperature of preferably between 0°C and 5°C and more preferably between 1°C and 3°C below the freezing point of said MCAA rich feed solution
c) Maintaining said MCAA rich feed solution at such under-cooling and adding seed crystals in an amount to prevent spontaneous nucleation which can turn said MCAA rich feed solution into an inseparable mass of billions of extremely fines crystals suspended in the remaining mother liquor.

For a crystallization process to be in balance, the rate of formed nuclei has to balance the number of crystals withdrawn from the system. The heat exchanger walls as required to withdraw heat in suspension based melt crystallization are typically scraped to prevent accumulation of the crystals in a crystal layer at the wall. Although the inventors were surprised to find that scraping is not a requirement and that the process can be performed with un-scraped heat exchangers, as well. A limitation is that, in case sufficient turbulence is applied, the driving temperature difference between the cooling medium and the MCAA suspension for a process without scraped surface heat exchangers has to be limited to a figure of about 5K. In case of insufficient turbulence this difference has to be lower. The inventors found that seeding is required to allow for a stable operation of a cooled vessel crystallizer without scraping where most probably insufficient nucleation rate in the crystallizer under such conditions is the reason for such phenomenon: when the rate at which crystals are withdrawn from the system is larger than the quantity of newly formed nuclei, the number of growing crystals in the vessel will gradually decrease; this in turn will eventually result in a sudden nucleation. While the effect is less pronounced than at the start of crystallization, it leads to a sudden decrease in the crystal size with related problems in the subsequent crystal liquid separation. The authors found that seed crystals in an amount of up to 5 % by weight and preferably between 0,1 and 1% by weight are advantageously added to the mixing vessel in order to avoid cycling of the crystal size and allow for stable long operation periods. Such seeds can be added continuously or intermittently preferably at intervals between 10 and 30 minutes without any significant influence on the crystal product quality.

While scraping of the heat exchanger surface is not required, it was unexpectedly found that scraping is an efficient means to avoid the continuous or intermittent addition of seed crystals. The combined cooling and scraping action at the crystallizer wall produces sufficient fines as to avoid such addition of seed crystals. Since the production of such seed crystals as well as the continuous or intermittent addition thereof is a complex and costly procedure it is a preferred embodiment of this invention to use a crystallizer with a scraped surface heat exchanger. Such a scraped surface heat exchanger can also be operated at a higher temperature difference between the product suspension and the cooling side, thereby requiring less heat exchanger surface to be installed.

Of course the required amount of energy to be discharged from the system to effect the crystallization can be effected by indirect cooling (e.g. jacketed vessels and heat exchangers), and/or by direct cooling (e.g. use of a coolant with or without phase transition of such coolant.

Very generally, the MCAA crystal suspension as produced according to the method of this invention can be separated by any solid-liquid device as known in the prior art. Such solid-liquid device may include, for example, filter, vacuum filter, pressure filter, centrifuges and the like. The large crystal surface area allowing the optimum growth conditions during preparation of the MCAA suspension, will negatively affect most attempts at separation. Impurities in the remaining liquid will adhere to all crystal surfaces and get included into the interstitial spaces of a compacted crystal cake: a highly efficient crystal-liquid separation is therefore required to completely remove these impurities and create a MCAA product according to required specification. Conventional mechanical separations require extensive washing to achieve even moderately pure product. In case molten product is used as washing liquid, about 10% to 20% of the crystallized product is lost and needs to be recycled to the crystallization process. In case a solvent is used as washing agent, such solvent would need to be recovered, which further increases complexity of the whole process. In any case the achievable purity of such separation is not sufficient to produce many of the technical grades MCAA. The washing provided by conventional separation systems is generally inefficient since the actual contact time between the wash liquid and the crystal is limited to only a few seconds. The contact time between wash liquid and crystal inside the packed bed of a wash column is much longer and generally in the range of 1-2 minutes. It was unexpectedly found that the much longer contact time found inside the wash column bed advantageously improves the wash efficiency and results in the higher purity MCAA produced from the wash column.

The present invention therefore proposes to separate the MCAA crystals from the remaining mother liquor in wash columns. According to the invention all wash columns which are mentioned in the prior art can be used. The publications Melt Crystallization - Fundamentals, Equipment and Applications edited by Joachim Ulrich, Shaker Verlag, 2003 and Melt Crystallization Technology; G.F. Arkenbout; Technomic Publishing Company Inc.; 1995 may be mentioned by way of example. Especially wash columns with forced transport as e.g. piston type wash columns, hydraulic wash columns or other continuous or semi continuous wash columns some of which are described in patents EP1427502B1, EP0920894B1, EP0097405B1, US3872009 and EP0083463B1 are particularly advantageous according to the invention and are included herein as reference.

The equipment for the method of this invention, especially the wash column and the crystallizer can be made of all types of construction materials typically used in corrosive applications, preferably plastics or metals or combination thereof. The crystallizer is preferably made of suitable alloys and/or glass lined materials of construction. In a specially preferred embodiment of the present invention the wash columns are constructed of plastic materials or metals coated with plastics or combinations thereof. Specifically suitable plastic materials are PVDF or PTFE. Although preferred embodiments of the present invention have been described in detail hereinabove, it should be clearly understood that many variations and/or modifications of the basic inventive concepts herein taught, which may appear to those skilled in the present art, will still fall within the spirit and scope of the present invention, as defined in the appended claims.

While tracing is essential to keep all equipment required for performing the method of the present invention free from encrustations and/or blockages, the inventors found that a sophisticated tracing along with a proper control of the tracing temperature around the crystal slurry containing components is crucial for the achievement of the purification results of the present invention. The inventors found that it is particularly useful to apply different tracing temperatures at the unwashed part of the wash column and at the opposite washed part of the wash column.

The novel procedure can also be used a number of times in series. It is particularly advantages to subject the residual mother liquor (C) from the purity crystallizer to a second crystallization step where more MCAA crystals are formed and to partially separate these crystals to form a MCAA enriched product that is combined with the initial MCAA rich starting mixture and a liquid residue that is discharged from the process. In this way the recovery of the total process can be further increased, while potentially maintaining the option to crystallize the MCAA product only once. Such a combined process is disclosed in EP1398064 B1, which is included herein by reference.

The invention will now be explained in greater detail herein below with reference to the non-limiting figures:
The suspension based melt crystallization system consists of four main components; (a) mixing vessel to suspend the crystals in mother liquor, (b) heat exchanger to discharge the amount of heat from the process as required for the formation of MCAA crystals. In a preferred embodiment of the present invention said heat exchanger features a scraping device (c) optionally an additional mixing vessel to optimize the mixed crystal flow and (d) a wash column for efficient removal of the crystals from the mother liquor. Components (a) and (b) may be combined into a single apparatus without any detrimental effects to the crystallization process.

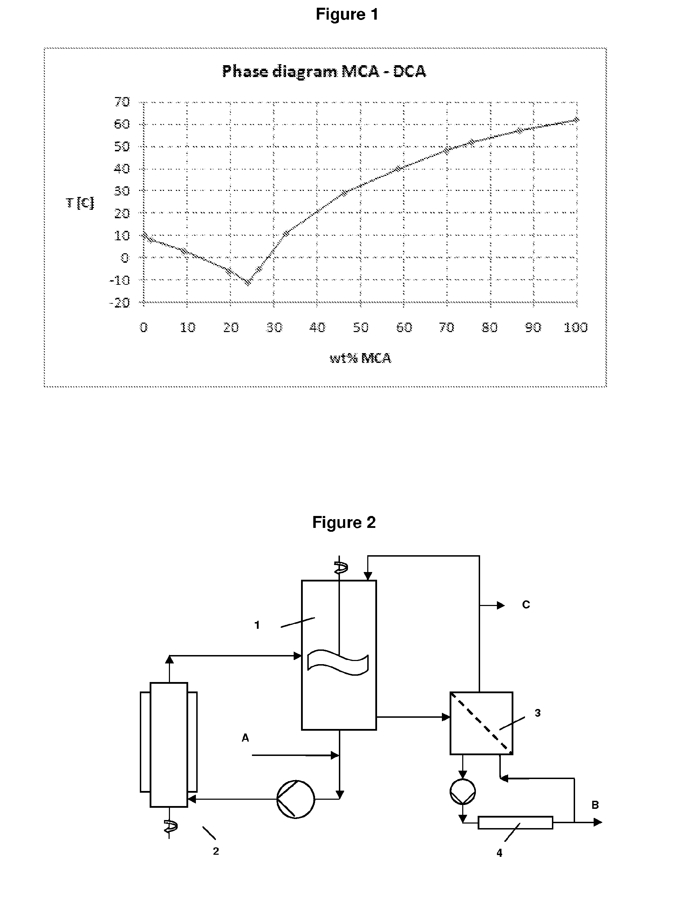

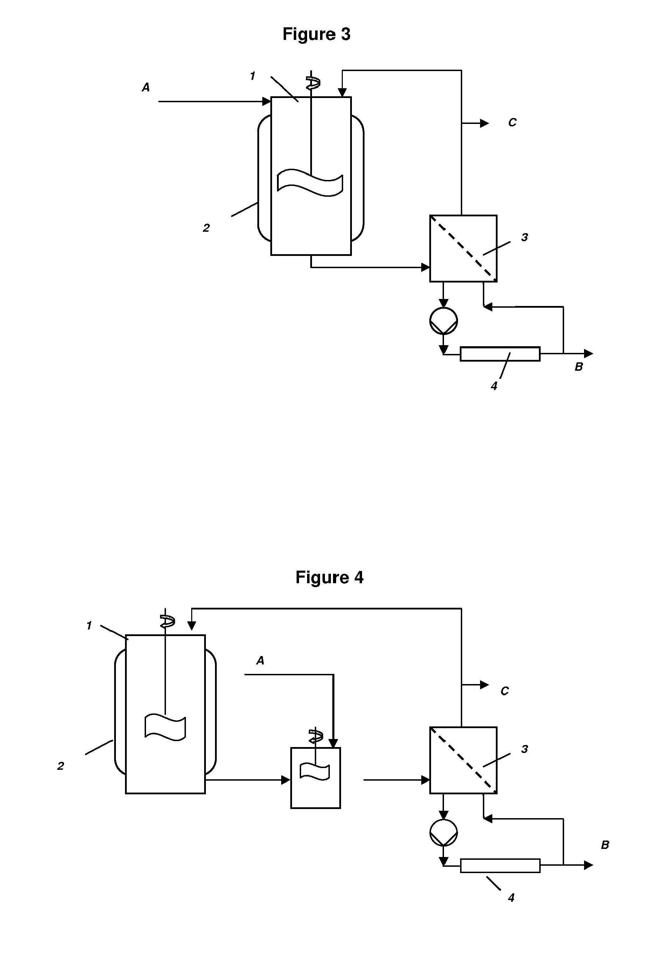

The specific process is described with reference to Figure 1. Figure 2 illustrates an equally advantageous configuration where components (a) and (b) are combined. The specific crystallization apparatus is not the intent of the invention and both systems are readily available in industry. Figure 3 illustrates the process with the additional mixing vessel mentioned in (c) above.

The MCAA rich feedstock (A) is fed to and circulated over the scraped surface heat exchanger crystallizer(s) where coolant is circulated in the outer jacket 2.

The scraped surface crystallizer(s) comprises a jacketed vessel containing a rotating scraper assembly to continuously prevent the cooled wall surface of being plugged with a crystal layer. The scraped surface crystallizer(s) are cooled by a secondary heat transfer liquid like e.g. water, brine, ethylene glycol solution or an evaporating refrigerant such as ammonia or Freon® in the outer jacket. Due to the freezing point of MCAA rich solutions, water can beneficially be used as cooling agent. The exact configuration is not critical and suitable systems are readily available in industry. The process heat is hereby removed from the system by a cooling system like air coolers in case of a secondary heat transfer liquid or a refrigeration system common in industry. The process equilibrium temperature in the scraped surface crystallizer(s) will typically range from +30°C to +55°C and preferably +40°C to +50°C. The mixing vessel(s) 1 are used to suspend the crystal slurry with typically 15% to 50% MCAA crystals by weight and preferably 25 to 35% crystals by weight in the mother liquor.

Surprisingly the intensity of the mixing is not critical and can be maintained at a very low rotational speed of 15 to 100 rpm or just enough to keep the crystal mass from settling to the bottom of the vessel. Again the specific design of the mixing vessel is not the intent of the invention and suitable apparatus are readily available in industry. The crystal volume in the mixing vessel should be sufficient to provide an average crystal residence time in the range of 15 minutes up to 2 hours and preferably in the range of 30 minutes to 1 hour. Preferably the residence time will not be extended beyond 1 hour since such longer periods will only marginally increase the crystal size. Excessively long residence time increase the complexity and cost of the required equipment. This amount of crystals provides a massive surface area for crystal growth. High growth surface areas result in very low growth rates. The very slow growth rates provided by suspension based crystallization ensure that the impurities that would normally be included in the rapidly growing crystal surface of a layer crystallization process are now excluded and remain in the mother liquor and results in pure MCAA product crystals. From the mixing vessel(s) the pure crystals suspended in impure mother liquor are fed to one or more wash column(s) 3. The wash column separates the crystals by compressing the slurry and allowing most of the mother liquor to leave through a filter to produce a thickened slurry in the form of a compact crystal bed with between 60% and 90% crystals by weight and preferably 65% to 75% by weight with the remainder being liquid mother liquor containing essentially all of the impurities. The compacted crystal bed is then forced through the column by hydraulic or mechanical force. The crystal bed is disintegrated and re-slurried opposite the original slurry entry point to form a pumpable new slurry in the melter loop (4).

The compact bed is composed of billions of individual crystals and thus forms a porous crystal cake mass within the wash column cylinder. The pressure in the melt loop is controlled by amount of product discharged in stream (B). The pressure in the melt loop 4 is thus controlled to a value higher than in the mixing vessel 1. The open space between the solid MCAA crystals is initially filled with impure mother liquor. Due to this pressure difference the melted product is forced counter-current to the crystal flow.

The crystals enter the wash column at the same temperature as the crystal slurry typically in the range from +20°C to +60°C and preferably +30°C to +53°C and most preferably +45 to +50°C. The purified MCAA product has melting temperature of +62°C. As the purified MCAA product flows thorough the porous crystal bed it will contact the colder crystal and the purified product will slowly re-crystallize onto the surface of the colder crystals. This recrystallization produces new crystalline MCAA product from the wash liquid. These new crystals are then carried out with the original crystal mass thus preventing the loss of valuable pure product to the process. The slow path of the wash liquid through the packed bed provides a relatively long contact between the wash liquid and crystal mass. This contact time can be controlled by the length of the crystal bed and throughput of the column. Since the pure wash liquid recrystallizes onto the surface of the existing crystals based on the same equilibrium driving forces found in the crystallizer the new crystals formed are extremely pure and this new crystal growth effectively drives the impurities from the crystal surface so that the impurities totally remain with the mother liquor. The wash column typically uses low pressure steam to melt the crystals in a heat exchanger 4 before discharging the purified product (B) but any other heat source can equally be used for such purpose.

Chloro acetic acid is usually marketed to the following specifications:
*Chloro acetic acid min. 99.0 wt%*
*Di chloro acetic acid max. 0.5 wt%*
*Acetic acid max. 0.2 wt%*
*Sulfate max. 0.5 wt%*
*Water max. 0.2 wt%*
*Iron max. 5 mg*/*kg*
*Lead max. 1 mg*/*kg*

Acid manufactured by the trichloroethylene method is virtually free of di chloro acetic acid; specially purified acid from the chlorination of acetic acid is marketed with an acetic acid content of max. 0.1 % and max. 0.3 % di chloro acetic acid. Various different technical grades contain up to 5 % di chloro acetic acid.

The results of the application of the invention are described in the examples below in more detail. The various applications of the invention shall, however, not be limited to such working examples, but may be varied with the accompanying claims.

### Example 1

An experiment was carried out in a cooled stirred glass beaker crystallizer which was filled with an amount of 800 ml of MCAA rich feed solution with a concentration of 91,5 wt.% of MCAA. The MCAA solution was first cooled to 54°C and the temperature was then maintained at that level for about 30 minutes. No formation of crystals could be observed. The temperature was then further reduced at a rate of 5°C per hour and after about 60 minutes and at a temperature of 50 °C spontaneous nucleation occurred, resulting in an inseparable mass of ultra-fine crystals and mother liquor.

### Example 2

The first part of the test of example 1 was repeated, but at this time approximately 10 grams of seed crystals were added at a temperature of 54°C. Instantly after the addition the temperature in the beaker vessel increased to 55.3°C, indicating the equilibrium temperature of the feed. The temperature in the vessel was then again reduced at a rate of 5°C per hour and after about 30 minutes the suspension as formed during this experiment was poured into a centrifuge in order to separate off the impurity containing mother liquor. A "dry" filter cake was achieved. Contrary to the test in Example 1, these crystals were easy to separate from the slurry.

### Example 3

In a dedicated test rig according to Figure 2 with a scraped surface vessel crystallizer and a mechanical piston column, an impure MCAA rich feed was added with a composition as further indicated in table 1.

**Table 1: Feed composition**

| **Component:** | **Unit** | **Value** |
|---|---|---|
| Monochloroacetic acid | **wt%** | **91,57** |
| Di chloro acetic acid | **wt%** | **5,90** |
| Tri chloro acetic acid | **wt%** | **0,02** |
| Acetic acid | **wt%** | **2,39** |
| Other impurities | **wt%** | **0,12** |

The scraped surface vessel crystallizer was cooled to a temperature of 53,5°C. The temperature was maintained at that level and seed crystals were added. Immediately after the seeding the temperature of the crystallizer increased to 55.,4°C. By further cooling down the cooling liquid which is circulated through the outer jacket, more crystals were formed. This was evidenced during the test by a continuous further decrease of the temperature in the crystallizer. The constant scraping of the crystallizer walls avoided the formation of any crystals at the heat exchanger surface. Approximately 3 hours after the formation of the first crystals the wash column was started.

The filtered mother liquor from the wash column was initially completely returned to the vessel crystallizer. In this way all impurities in the system kept on accumulating and the concentration of MCAA decreased continuously. This could be observed by a simultaneous decrease of the temperature in the crystallizer, which represents the equilibrium temperature of the MCAA solution in the crystallizer at a certain amount of impurities. After an MCAA concentration of about 84 wt.% was reached a certain amount of reject was discharged from the process to keep the impurity concentration in the process constant. The amount of reject from the process was controlled such as to keep the crystallizer temperature constant. The liquid fraction as rejected from the process via the filter of the wash column had a composition as indicated as example in Table 2.

**Table 2: Reject composition**

| **Component:** | **Unit** | **Value** |
|---|---|---|
| Monochloroacetic acid | **wt%** | **83,6** |
| Di chloro acetic acid | **wt%** | **10,90** |
| Tri chloro acetic acid | **wt%** | - |
| Acetic acid | **wt%** | **5,50** |
| Other impurities | **wt%** | - |
| | | |

The suspension from the crystallizer was compacted in the wash-column into a dense crystal bed. At the bottom side such packed crystal bed was disintegrated by means of a slowly rotating scraping knife and the individual crystal were re-suspended in re-circulating molten product and then molten in the wash-column melter. A part of this pure product stream was returned into the wash column to achieve the efficient counter-current washing of the crystal bed, while the reminder was discharged from the process as pure product. The wash column purification could be observed visually, indicated by an increasingly clear appearance of the pure product samples compared to the dark color of the feed and the reject.

As an example for the attained product purities, Table 3 shows a product composition as sampled about 10 to 12 hours after the start of the crystallization.

**Table 3: Product composition**

| **Component:** | **Unit** | **Value** |
|---|---|---|
| Monochloroacetic acid | **wt%** | **99,9** |
| Di chloro acetic acid | **wt%** | **0,1** |
| Tri chloro acetic acid | **wt%** | - |
| Acetic acid | **wt%** | - |
| Other impurities | **wt%** | - |

These results prove that the method of the invention is suitable to produce MCAA with the required purity. In particular the produced MCAA complied with the pure product specifications for di chloro acetic acid and acetic acid.

## Claims

1. A method for the recovery of purified MCAA from a crude feed stream containing said MCAA, said method comprising the steps of:
a. adding the MCAA rich feed solution to a mixing vessel and cooling down said MCAA containing feed solution to a temperature of preferably between 0 °C and 10°C, more preferably between 1°C and 5°C, and most preferably between 1°C and 2°C below the equilibrium temperature of said MCAA containing feed solution,
b. maintaining said MCAA containing feed solution at such under-cooling and optionally adding seed crystals in an amount to prevent spontaneous nucleation to form a slurry containing solid MCAA crystals,
c. further cooling said slurry to create a volume with a crystal content of preferably between 10% and 40% by weight of the MCAA crystals and more preferably between 20% and 35%, wherein the crystals are grown in suspension from a slightly under-cooled melt and are not fixed onto an internal crystallizer surface, the available surface for crystal growth being not less than 5,000 m² per m³ of crystallizer volume,
d. holding said slurry in a mixing vessel to create a crystal residence time preferably between 15 minutes and 4 hours, more preferably between 30 minutes and 2 hours and most preferably of about 1 hour,
e. separating said MCAA crystals from the mother liquor in a suitable separation device.
f. discharging the separated crystals as purified MCAA product and
g. discharging the separated mother liquor as separate product

2. Method according to claim 1, wherein step e is carried out using a wash column with forced transport of the compacted crystal bed, preferably a piston type wash column or a hydraulic wash column , to separate the said MCAA crystals from the mother liquor, in which a portion of the MCAA crystals is heated until they are melted to form a liquid of the purified MCAA, and in which the MCAA crystals are washed as they are forced through the wash column, by contact with the purified melt of said crystals being forced through the crystal cake.

3. Method according to claim 1 or 2 where in step b) the addition of seed crystals is omitted.

4. Method according to claim 1 or 2, wherein seed crystals in an amount of preferably up to 5 % by weight and more preferably between 0,1 and 1% by weight are added to the mixing vessel.

5. Method according to any of the previous claims, , wherein the cooling surface is scraped to keep the cooling surface free from incrustations and wherein scraper blades produce such an amount of fines forming seed crystals for preventing spontaneous nucleation.

6. Method according to any of the previous claims, , wherein the process is carried out using separate apparatus for at least one crystal producing step and at least one crystal conditioning step

7. Method according to any of the previous claims, , wherein the process is carried out using at least one single apparatus for a combined crystal producing step and crystal conditioning step.

8. Method according to claim 6 or 7, wherein an additional mixing vessel is used to optimize the mixed crystal flow to the wash column(s) for efficient removal of the MCAA crystals from the mother liquor.

9. Method according to any of the claims 2-8, wherein the wash column is constructed or predominantly constructed from plastic material or metals coated with plastic.

10. MCAA obtained with the method according to any of the claims 1-9.
